# EUROPÄISCHE PATENTSCHRIFT

(11) **EP 0 398 155 B1**
(45) Veröffentlichungstag und Bekanntmachung des Hinweises auf die Patenterteilung: **07.02.1996**
(21) Anmeldenummer: 90108802.1
(22) Anmeldetag: 10.05.1990
(51) Int. Cl.: C07D 239/26, C09K 19/34, C09K 19/30, C07D 239/34, C07C 69/92, C07D 213/30, C07C 43/205, C07C 43/184

(54) **Cyclopropylalkyl oder -alkenylverbindungen, Verfahren zu ihrer Herstellung und ihre Verwendung in flüssigkristallinen Mischungen**
Cyclopropylalkyl or cycloalkenyl compounds, process for their preparation and their use in liquid crystal mixtures
Composés de cyclopropylalcoyle ou cyclopropylalcényle, procédé pour leur préparation et leur utilisation dans des mélanges de cristaux liquides

(30) Priorität: 13.05.1989 DE 3915804
(43) Veröffentlichungstag der Anmeldung: 22.11.1990
(73) Patentinhaber: HOECHST AKTIENGESELLSCHAFT, D-65926 Frankfurt am Main (DE)
(72) Erfinder: de Meijere, Armin, Prof. Dr., D-3400 Göttingen, (DE); Dübal, Hans-Rolf, Dr., D-6240 Königstein/Taunus (DE); Escher, Claus, Dr., D-6109 Mühltal (DE); Harada, Takamasa, Dr.M.Sc., D-6370 Oberursel, (DE); Hemmerling, Wolfgang, Dr., D-6231 Sulzbach(Taunus) (DE); Illian, Gerhard, Dr., D-6230 Frankfurt am Main (DE); Müller, Ingrid, Dr., D-6238 Hofheim am Taunus (DE); Murakami, Mikio, Kakegawa-shi Skizuka-ken (JP); Ohlendorf, Dieter, Dr., D-6237 Liederbach (DE); Wingen, Rainer, Dr., D-6234 Hattersheim am Main (DE)

(56) Entgegenhaltungen:
- EP-A- 0 244 129
- EP-A- 0 318 423

## Beschreibung

Die ungewöhnliche Kombination von anisotropem und fluidem Verhalten der Flüssigkristalle hat zu ihrer Verwendung in einer Vielzahl von elektro-optischen Schalt- und Anzeigevorrichtungen geführt. Dabei können ihre elektrischen, magnetischen, elastischen und/oder thermischen Eigenschaften zu Orientierungsänderungen benutzt werden. Optische Effekte lassen sich dann beispielsweise mit Hilfe der Doppelbrechung, der Einlagerung dichroitisch absorbierender Farbstoffmoleküle ("guest-host mode") oder der Lichtstreuung erzielen.

Zur Erfüllung der ständig steigenden Praxisanforderungen auf den verschiedenen Anwendungsgebieten besteht laufend ein Bedarf an neuen verbesserten Flüssigkristall("liquid crystal")-Mischungen und somit auch an einer Vielzahl mesogener Verbindungen unterschiedlichster Struktur. Dies gilt sowohl für die Gebiete, bei denen nematische LC-Phasen (z. B. TN = "twisted nematic", STN = "supertwisted nematic", SBE = "supertwisted birefringence effect", ECB = "electrically controlled birefringence") verwendet werden, als auch für solche mit smektischen LC-Phasen (z. B. ferroelektrische, elektrokline).

Viele der für LC-Mischungen geeigneten Verbindungen lassen sich durch ein Aufbauprinzip (Bauschema) beschreiben [siehe z. B. J. Am. Chem. Soc. 108, 4736 (1986), Struktur I; Science 231, 350 (1986), Fig. 1 A; J. Am. Chem. Soc. 108, 5210 (1986), Fig. 3], bei dem Kerne aus cyclischen Verbindungen - Aromaten, Heteroaromaten, aber auch gesättigte Ringsysteme - mit geradkettigen oder in der Kette durch kleine Gruppen (z. B. Methyl, Chlor) substituierten und somit verzweigten Alkylseitenketten verknüpft sind.

In der EP-A 0 244 129 werden 2,2-Dimethylcyclopropan-Derivate beschrieben, die optisch-aktiv sind und über eine -CO-O-CH₂, -O-CO- oder -CH₂-Brücke (am rechten Ende des jeweiligen Brückenglieds befindet sich der Cyclopropylring) mit einem der bekannten mesogenen Reste verbunden sind. Diese Verbindungen sollen als Komponenten für ferroelektrische LC-Mischungen (spontane Polarisation bis zu 11 nC/cm²) geeignet sein.

Aufgäbe der vorliegenden Erfindung ist es, neue mesogene Verbindungen zur Verfügung zu stellen, die mit vielen anderen Komponenten zu unterschiedlichsten LC-Mischungen kombiniert werden können. Die nachstehend definierten Verbindungen lösen diese Aufgabe (Die in der nachpublizierten EP-A 0 318 423 offenbarten Verbindungen sind vom vorliegenden Anspruchsumfang mittels Disclaimer ausgenommen):

Flüssigkristalline Cyclopropylalkyl- oder -alkenyl-Verbindungen der allgemeinen Formel (I) 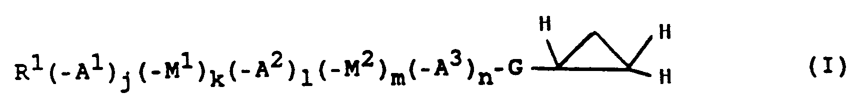
in der bedeuten:
- R¹: geradkettiges oder verzweigtes (mit oder ohne asymmetrisches C-Atom) Alkyl oder Alkenyl mit 2 bis 16 C-Atomen, wobei auch eine oder zwei nicht-benachbarte -CH₂-Gruppen durch -O-, -S-, -CO-, -CO-O-, -O-CO- oder -O-CO-O- ersetzt sein können und wobei auch H durch F ersetzt sein kann,
oder einer der nachfolgenden Reste 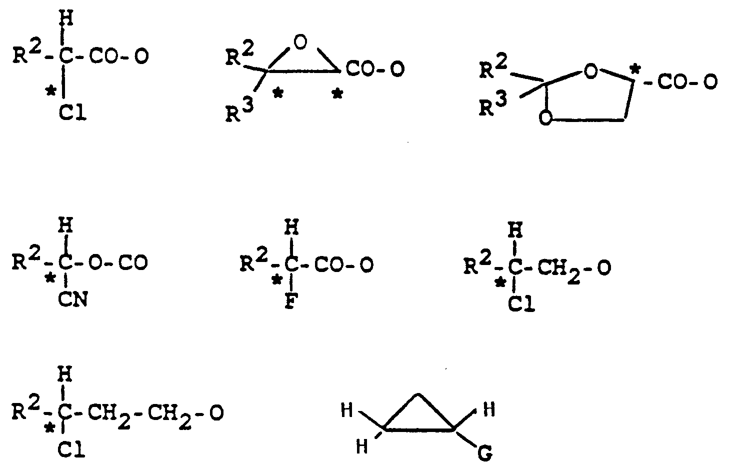 CN, OCF₃, OCF₂H, F, CF₃
- A¹,A²,A³: gleich oder verschieden unsubstituiertes oder mit 1 oder 2 Halogenatomen substituiertes 1,4-Phenylen, unsubstituiertes oder in 1 oder 4 Stellung mit CN substituiertes 1,4-Cyclohexylen, Pyrazin-2,5-diyl, Pyridazin-3,6-diyl, Pyridin-2,5-diyl, Pyrimidin-2,5-diyl, (1,3,4)-Thiadiazol-2,5-diyl
- M¹,M²: gleich oder verschieden CO-O, O-CO, CO-S, S-CO, CH₂-O, O-CH₂, C≡C, CH₂-CH₂ oder eine Einfachbindung
- G: geradkettiges oder verzweigtes Alkylen mit 1 bis 16 C-Atomen oder Alkenylen mit 2 bis 16 C-Atomen, bei dem auch eine oder zwei nicht-benachbarte -CH₂-Gruppen durch -O-, -S-, -O-CO-, -CO-O-, -S-CO- oder -CO-S- ersetzt sein können
- R²,R³,: H oder geradkettiges oder verzweigtes Alkyl mit 1 bis 16 oder Alkenyl mit 2 bis 16 C-Atomen, bei dem auch eine -CH₂-Gruppe durch -O-, -CO-O- oder -O-CO- ersetzt sein kann
- k,m,: Null oder 1
- j,l,n,: Null, 1 oder 2
Bevorzugt sind solche Verbindungen, bei denen in der allgemeinen Formel (I) die Gruppierung (-A¹)ⱼ(-M¹)ₖ(-A²)ₗ(-M²)ₘ(-A³)ₙ- bedeutet: 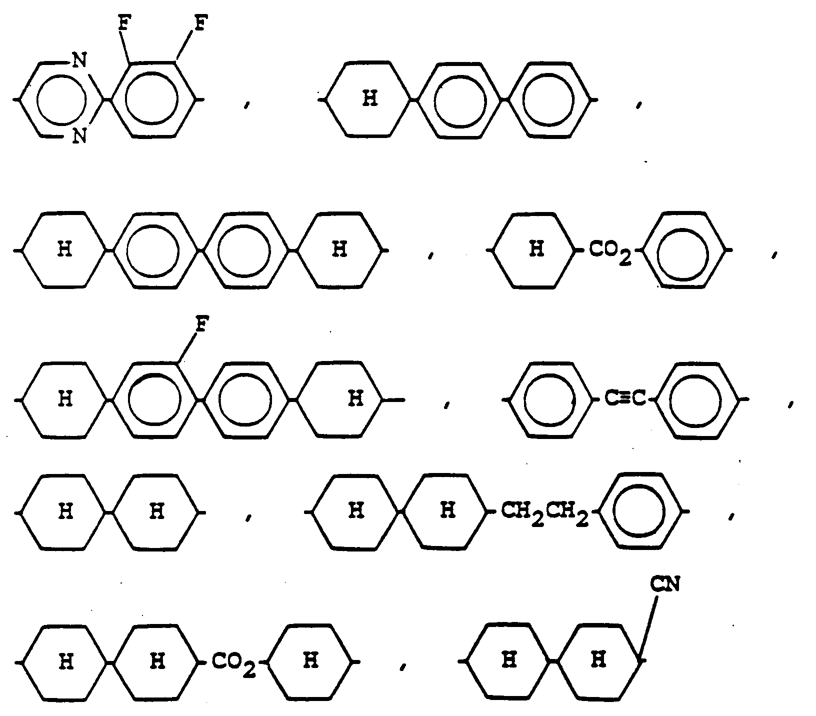 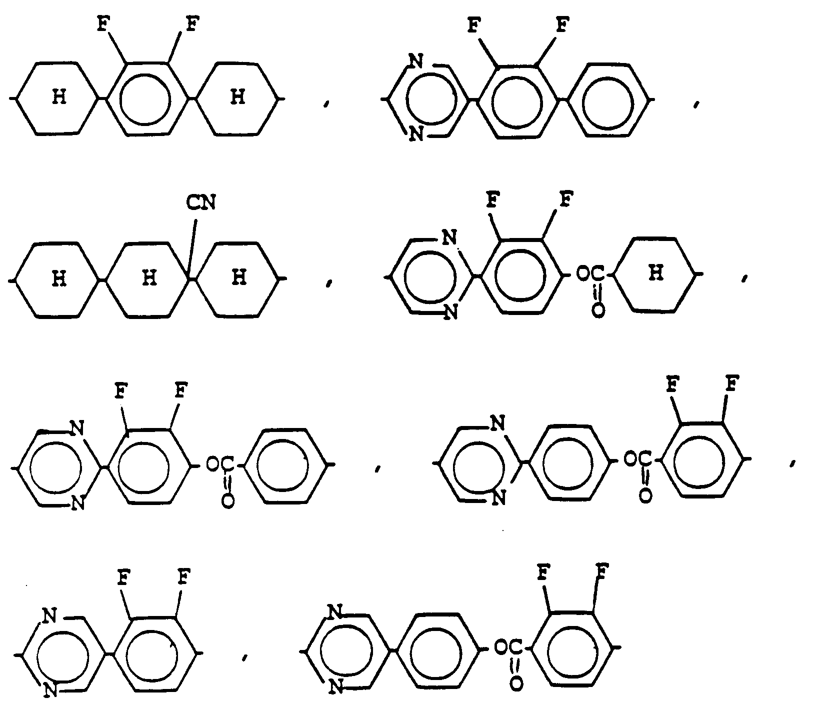
Weiterhin sind besonders bevorzugt Verbindungen, bei denen in der allgemeinen Formel (I) die Gruppierung R¹(A-¹)ⱼ (-M¹)ₖ (-A²)ₗ (-M²)ₘ (-A³)ₙ bedeutet: 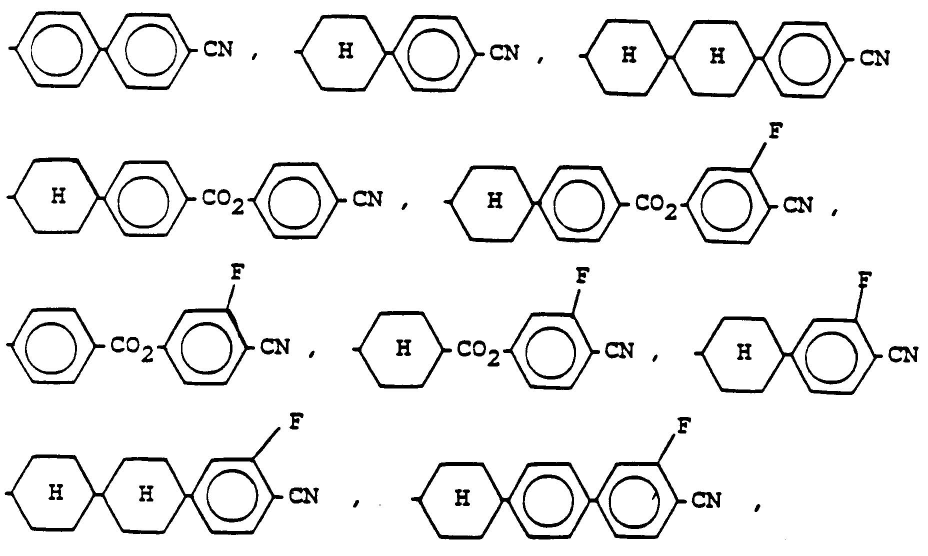 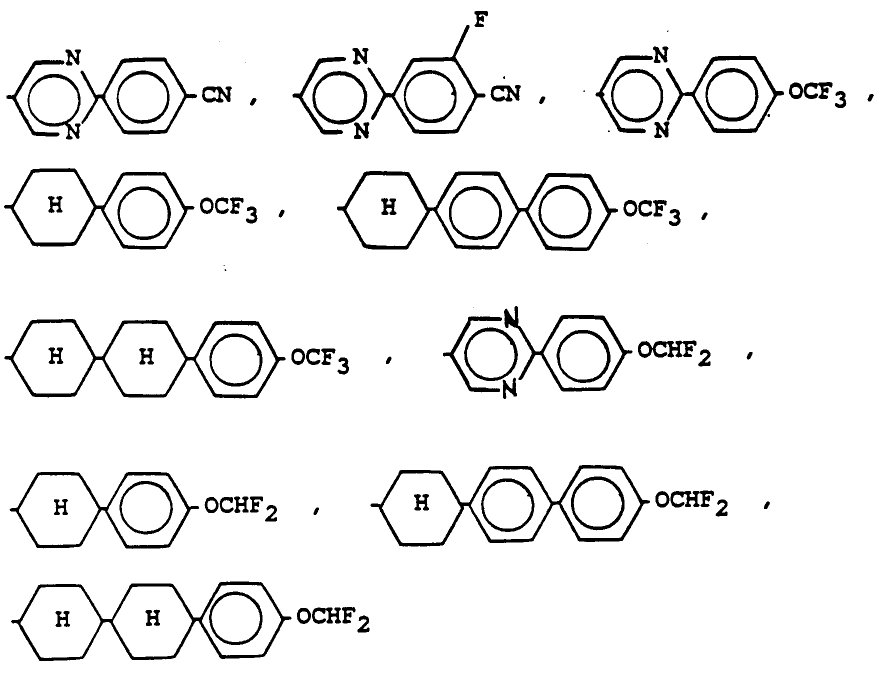
Besonders bevorzugt werden auch Verbindungen der allgemeinen Formel (I), bei denen A¹, A² und A³ gleich oder verschieden unsubstituiertes 1,4-Phenylen, mit 1 oder 2 Fluoratomen substituiertes 1,4-Phenylen, 1,4-Cyclohexylen, Pyridin-2,5-diyl oder Pyrimidin-2,5-diyl bedeuten.

Die neuen Cyclopropylalkyl- oder -alkenylverbindungen sind chemisch, photochemisch und thermisch stabil und verfügen über eine gute Mischungskompatibilität. Im Vergleich zu den entsprechenden n-Alkyl-Homologen weisen diese Verbindungen oftmals einen niedrigeren Schmelzpunkt auf und führen in LC-Mischungen häufig zu niedrigeren Schmelzpunkten.

Eine weitere Lösung der gestellten Aufgabe ist eine Flüssigkristall-Mischung mit einem Gehalt an mindestens einer flüssigkristallinen Verbindung, der allgemeinen Formel (I).

Die Flüssigkristall-Mischungen bestehen aus 2 bis 20, vorzugsweise 2 bis 15 Komponenten, darunter mindestens einer der erfindungsgemäß beanspruchten Verbindungen. Die anderen Bestandteile werden vorzugsweise ausgewählt aus den bekannten Verbindungen mit nematischen, cholesterischen und/oder geneigt-smektischen Phasen, dazu gehören beispielsweise Schiffsche Basen, Biphenyle, Terphenyle, Phenylcyclohexane, Cyclohexylbiphenyle, Pyrimidine, Zimtsäureester, Cholesterinester, verschieden überbrückte, terminal-polare mehrkernige Ester von p-Alkylbenzoesäuren. Im allgemeinen liegen die im Handel erhältlichen Flüssigkristall-Mischungen bereits vor der Zugabe der erfindungsgemäßen Verbindung(en) als Gemische verschiedenster Komponenten vor, von denen mindestens eine mesogen ist, d. h. als Verbindung, in derivatisierter Form oder im Gemisch mit bestimmten Cokomponenten eine Flüssigkristall-Phase zeigt [= mindestens eine enantiotrope (Klärtemperatur > Schmelztemperatur) oder monotrope (Klärtemperatur < Schmelztemperatur) Mesophasenbildung erwarten läßt].

Von der oder den erfindungsgemäßen Verbindung(en) enthalten die Flüssigkristall-Mischungen im allgemeinen 0,01 bis 70 Gew.-%, insbesondere 0,05 bis 50 Gew.-%.

Die erfindungsgemäßen Verbindungen können nach an sich bekannten Standardreaktionen aus mesogenen monofunktionellreaktionsfähigen Grundkörpern durch Verknüpfung mit ebenfalls monofunktionell-reaktionsfähigen Cyclopropylalkyl-Verbindungen hergestellt werden, wobei die Synthese beider Komponenten als bekannt vorausgesetzt werden kann.

So können beispielsweise mesogene Hydroxy- oder Mercapto-Verbindungen mit Cyclopropyl-alkanolen in Anwesenheit von Triphenylphosphin/Azodicarbonsäurediester (Mitsunobu-Reaktion, z. B. in J. Chem. Soc. Perkin Trans. 1975, 461) verknüpft werden. Es können auch die separat oder intermediär erzeugten Alkali- oder Erdalkalisalze dieser mesogenen Hydroxy- oder Mercapto-Verbindungen mit Halogen-, Toluolsulfonyloxy- oder Methylsulfonyloxy-cyclopropylalkyl-Verbindungen umgesetzt werden (Williamson-Reaktion, z. B. in Patai, The Chemistry of the Ether Linkage, Interscience Publishers, Neu York 1967, S. 446 - 468).

Es können aber auch mesogene Carbonsäuren mit Cyclopropylalkanolen unter Kondensationsbedingungen (z. B. in March, Advanced Organic Chemistry, 2nd Ed., Mc. Graw-Hill Kogakuska Ltd., Tokyo 1977, S. 363 - 365) umgesetzt werden. In gleicher Weise ist dies auch mit mesogenen Hydroxy- oder Mercapto-Verbindungen und Cyclopropyl-alkansäuren möglich.

Die zur Verknüpfung erforderlichen Cyclopropylalkyl-Verbindungen werden nach Standardmethoden hergestellt, es wird dazu auf die vorstehend erwähnten Veröffentlichungen (US-A) von Henrick et al. verwiesen.

Weiterhin können Cyclopropylverbindungen durch die Simmons-Smith-Reaktion (siehe z.B. in March Advanced Organic Chemistry s. 793 - 794) aus den entsprechenden Olefinen hergestellt werden.

### Beispiel 1

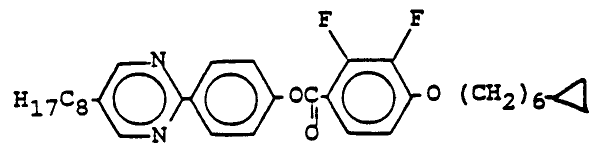

### 4-(6-Cyclopropylhexyloxy)-2,3-difluor-benzoesäure-[4-(5-octylpyrimidin-2-yl)phenyl]ester

### Herstellung von 4-(6-Cyclopropylhexyloxy)-2,3-difluorbenzoesäure:

Zu einer Lösung von 8,5 g Difluorphenol in 100 ml DMF werden 3,9 g NaH (60 % in Öl) und 20 g 6-Cyclopropylhexanol gegeben. Nach 24 h Reaktionszeit bei Raumtemperatur wird die Lösung auf Eis gegossen und mit CH₂Cl₂ extrahiert. Nach Trocknen (Na₂SO₄) und Abdestillieren des Lösungsmittels werden 16,5 g 6-Cyclopropylhexyloxy -2,3-difluor-benzol als farbloses Öl erhalten.

Zu einer Lösung von 11,7 g 6-Cyclopropylhexyloxy-2,3-difluor-benzol in 70 ml THF werden bei -60°C 30 ml Butyllithium (1,6 m in Hexan) getropft. Nach 5 h bei -60°C wird CO₂ bis zur Sättigung eingeleitet. Die Lösung wird auf RT erwärmt, das Lösungsmittel abdestilliert, mit 400 ml H₂O versetzt und mit Essigsäure auf pH 3 angesäuert. Der Niederschlag wird abgesaugt. Erhalten werden 12,3 g 4-(6-Cyclopropylhexyloxy)-2,3-difluor-benzoesäure. Schmp. 100-108°C
Analog werden erhalten:
4-(4-Cyclopropylbutyloxy)-2,3-difluor-benzoesäure
4-(8-Cyclopropyloctyloxy)-2,3-difluor-benzoesäure
Zu einer Lösung von 0,84 g Dicyclohexylcarbodiimid 50 mg Dimethylaminopyridin und 1,15 g 4-(5-Octylpyrimidin-2-yl)phenol in 50 ml Methylenchlorid werden 1,2 g 4-(6-Cyclopropylhexyloxy)-2,3-difluor-benzoesäure gegeben. Nach einer Reaktionszeit von 24 h wird der Niederschlag (Dicyclohexylharnstoff) abgesaugt. Das Filtrat wird vom Lösungsmittel befreit und chromatrographiert (SiO₂, CH₂Cl₂).

Nach Umkristallisation aus Hexan erhält man 1,1 g 4-(6-Cyclopropylhexyloxy)-2,3-difluor-benzoesäure-[4-(5-octyl-pyrimidin-2-yl)phenyl]ester.

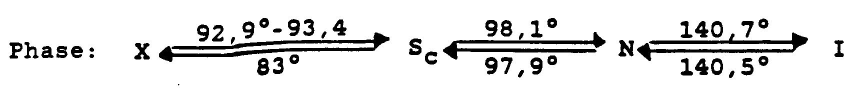

### Analog werden erhalten:

### Beispiel 2

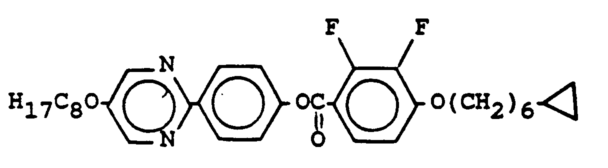

4-(6-Cyclopropylhexyloxy)-2,3-difluor-benzoesäure-[4-(5-octyloxy-pyrimidin-2-yl)phenyl]ester

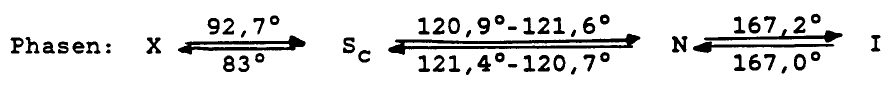

### Beispiel 3

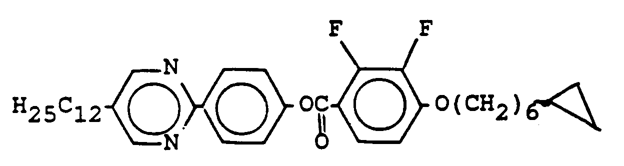

4-(6-Cyclopropylhexyloxy)-2,3-difluor-benzoesäure-[4-(5-dodecyl-pyrimidin-2-yl)phenyl]ester

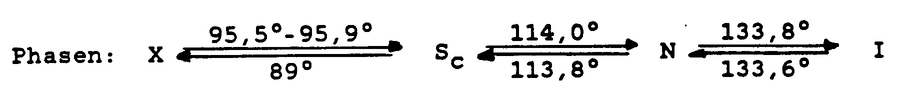

### Beispiel 4

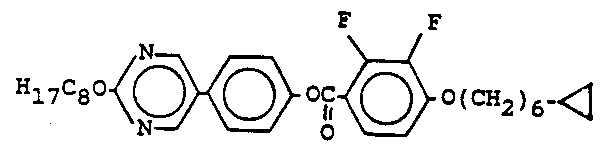

4-(6-Cyclopropylhexyloxy)-2,3-difluor-benzoesäure[4-(2-octyloxy-pyrimidin-5-yl)phenyl]ester

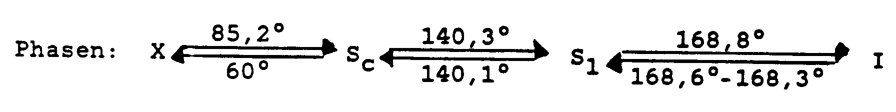

### Beispiel 5

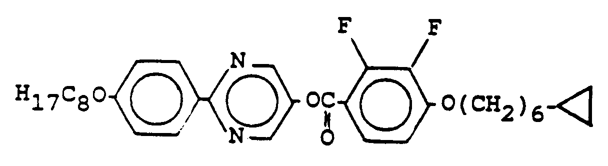

4-(6-Cyclopropylhexyloxy)-2,3-difluor-benzoesäure[2-(4-octyloxyphenyl)-pyrimidin-5-yl]ester

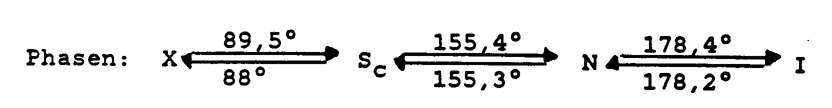

### Beispiel 6

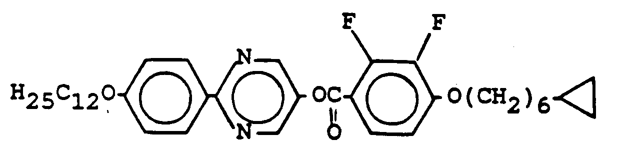

4-(6-Cyclopropylhexyloxy)-2,3-difluor-benzoesäure[2-(4-dodecyloxyphenyl)-pyrimidin-5-yl]ester

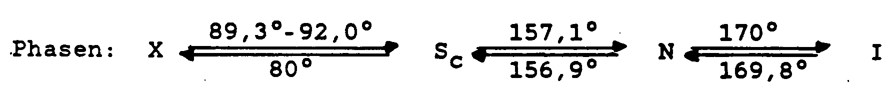

### Beispiel 7

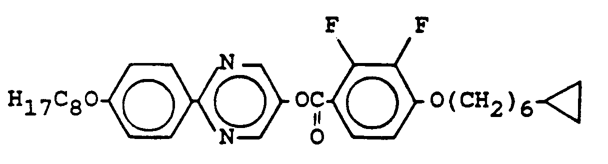

4-(6-Cyclopropylhexyloxy)-2,3-difluor-benzoesäure[2-(4-octyloxyphenyl)-pyrimidin-5-yl]ester

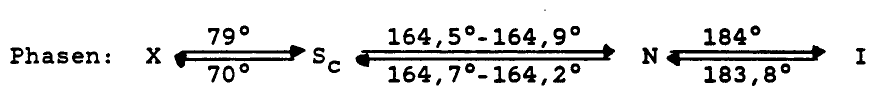

### Beispiel 8

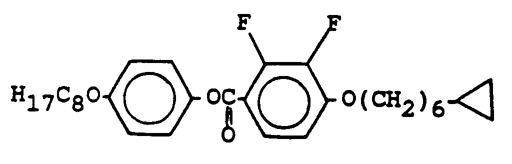

4-(6-Cyclopropylhexyloxy)-2,3-difluor-bensoesäure(4-octyloxy)phenylester

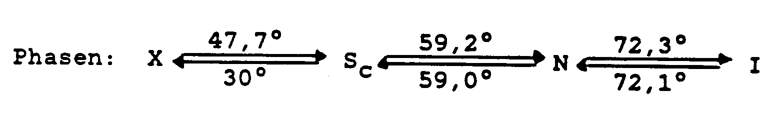

### Vergleichsbeispiel

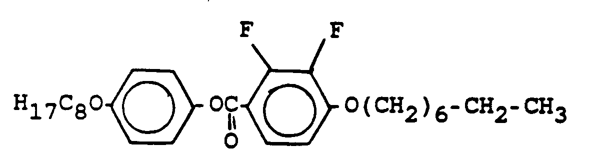

4-Octyloxy-2,3-difluor-benzoesäure-(4-octyloxy)phenylester 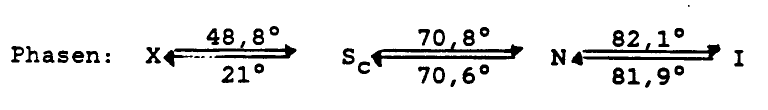
Der Vergleich der erfindungsgemäßen Substanz aus Beispiel 8 zu der genannten Verbindung zeigt, daß die erfindungsgemäße Verbindung einen niedrigeren Schmelzpunkt besitzt.

Der Ersatz der Ethylgruppe durch einen Cyclopropylrest bewirkt eine Erniedrigung des Schmelzpunktes um ca. 1 Grad.

### Beispiel 9

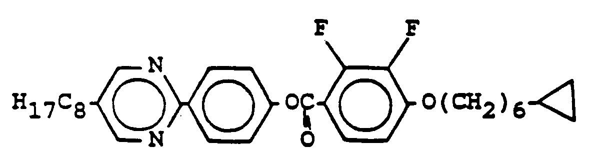

4-(6-Cyclopropylhexyloxy)-2,3-difluor-benzoesäure[4-(5-octyl-pyrimidin-2-yl)phenyl]ester

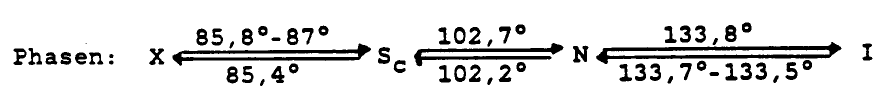

### Beispiel 10

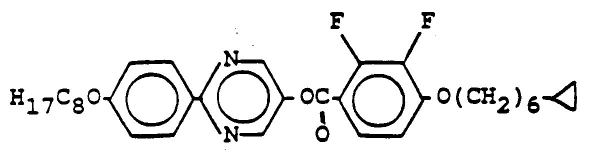

4-(6-Cyclopropylhexyloxy)-2,3-difluor-benzoesäure[4-(5-octyloxy-pyrimidin-2-yl)phenyl]ester

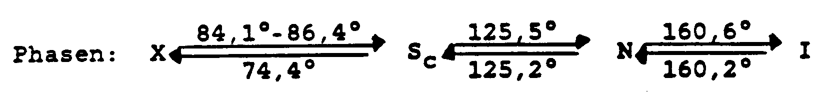

### Beispiel 11

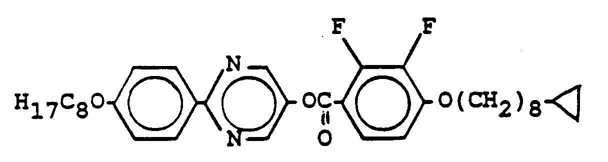

4-(8-Cyclopropyloctyloxy)-2,3-difluor-benzoesäure[4-(5-octyloxyphenyl)pyrimidin-5-yl]ester

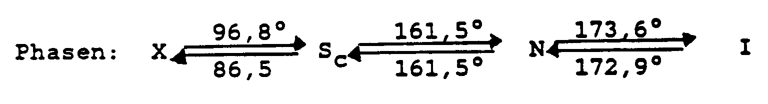

### Beispiel 12

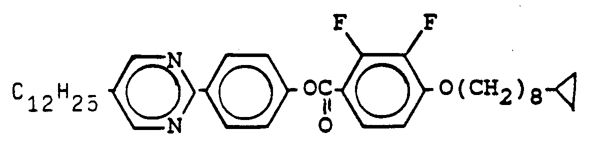

4-(8-Cyclopropyloctyloxy)-2,3-difluor-benzoesäure[4-(5-dodecyl-pyrimidin-2-yl)phenyl]ester

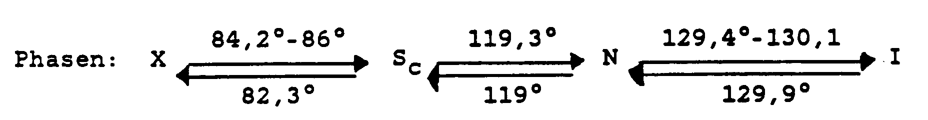

### Beispiel 13

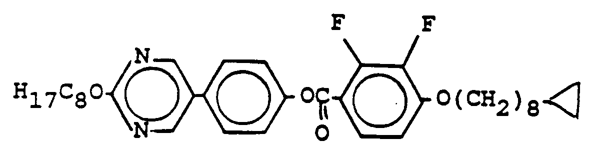

4-(8-Cyclopropyloctyloxy)-2,3-difluor-benzoesäure[4-(2-octyloxy-pyrimidin-5-yl)phenyl]ester

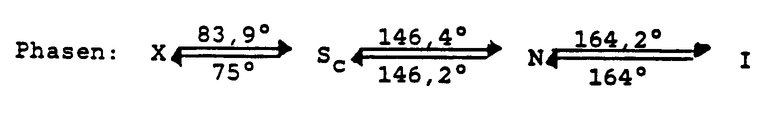

### Beispiel 14

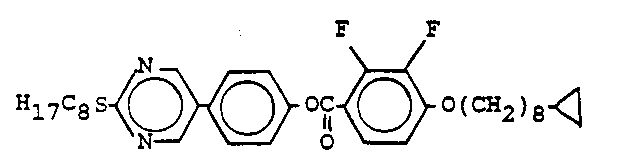

4-(8-Cyclopropyloctyloxy)-2,3-difluor-benzoesäure[4-(2-octylthio-pyrimidin-5-yl)phenyl]ester

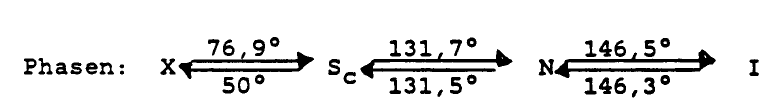

### Beispiel 15

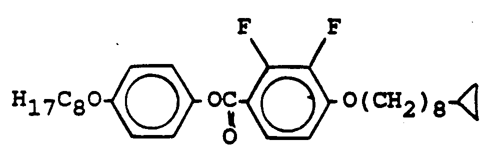

4-(8-Cyclopropyloctyloxy)-2,3-difluor-benzoesäure-(4-octyloxy)-phenylester

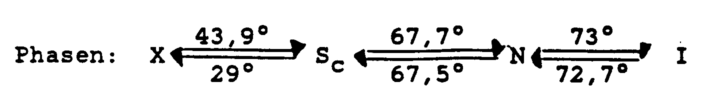

### Vergleichsbeispiel

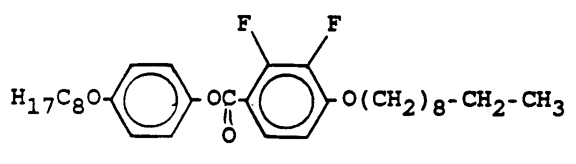

4-Octyloxy-2,3-difluor-benzoesäure-(4-decycloxy)-phenylester 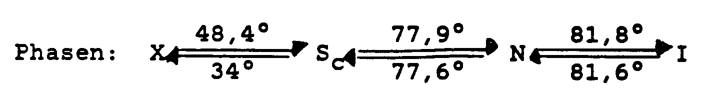
Die erfindungsgemäße Verbindung aus Beispiel 15 weist einen um etwa 5 Grad niedrigeren Schmelzpunkt auf als die Vergleichsverbindung. Der Ersatz einer Ethylgruppe durch einen Cyclopropylrest führt zu dieser für praktische Anwendungen günstigen Schmelpunktserniedrigung.

### Beispiel 16

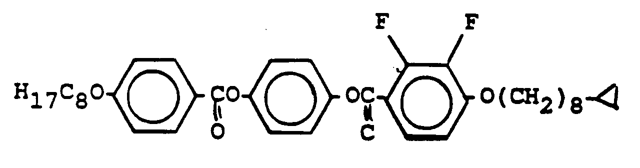

4-(8-Cyclopropyloctyloxy)-2,3-difluor-benzoesäure[4-(4-octyloxy-benzoyloxy)phenyl]ester

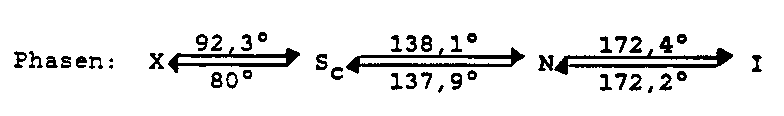

### Beispiel 17

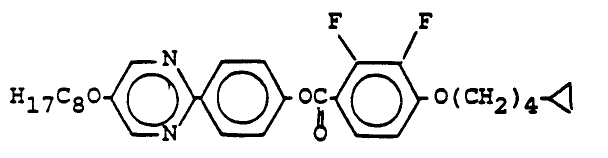

4-(4-Cyclopropylbutyloxy)-2,3-difluor-benzoesäure[4-(5-octyloxy-pyrimidin-2-yl)phenyl]ester

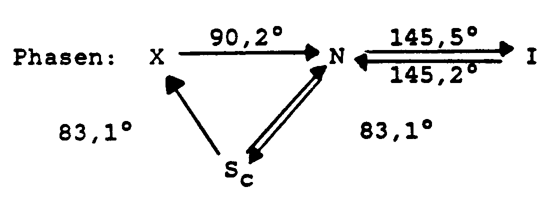

### Beispiel 18

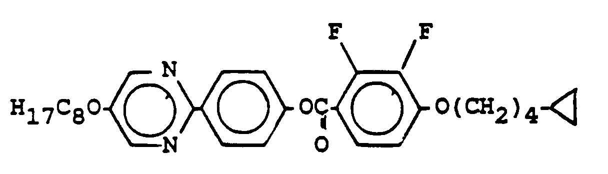

4-(4-Cyclopropylbutyloxy)-2,3-difluor-benzoesäure[4-(5-octyloxy-pyrimidin-2-yl)phenyl]ester

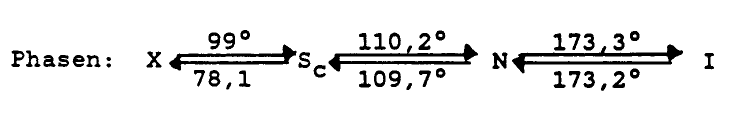

### Beispiel 19

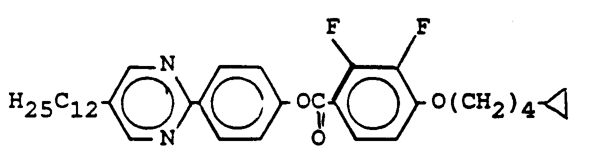

4-(4-Cyclopropylbutyloxy)-2,3-difluor-benzoesäure[4-(5-dodecyl-pyrimidin-2-yl)phenyl]ester

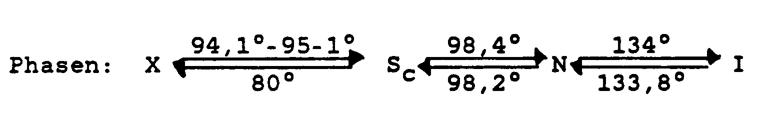

### Beispiel 20

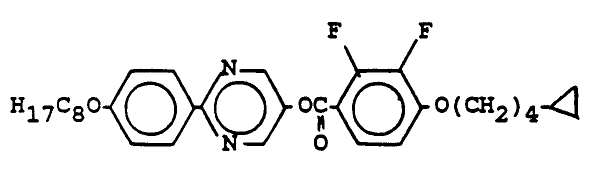

4-(4-Cyclopropylbutyloxy)-2,3-difluor-benzoesäure[2-(4-octyloxyphenyl)pyrimidin-5-yl]ester

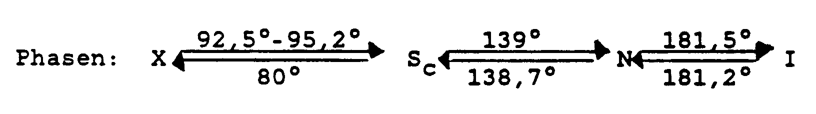

### Beispiel 21

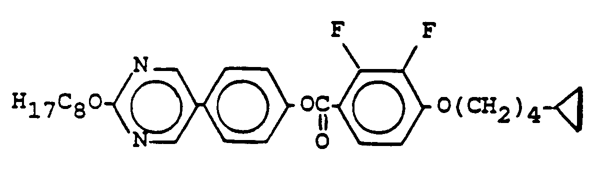

4-(4-Cyclopropylbutyloxy)-2,3-difluor-benzoesäure[4-(2-octyloxy-pyrimidin-5-yl)phenyl]ester

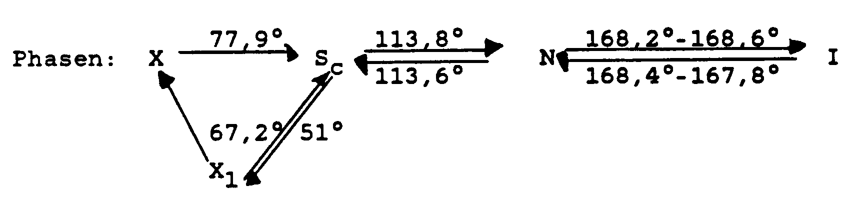

### Beispiel 22

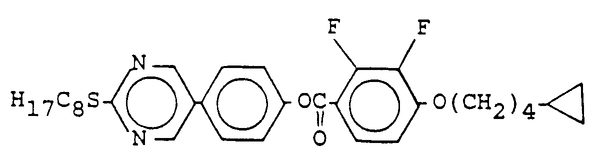

4-(4-Cyclopropylbutyloxy)-2,3-difluor-benzoesäure[4-(2-octylthio-pyrimidin-5-yl)phenyl]ester

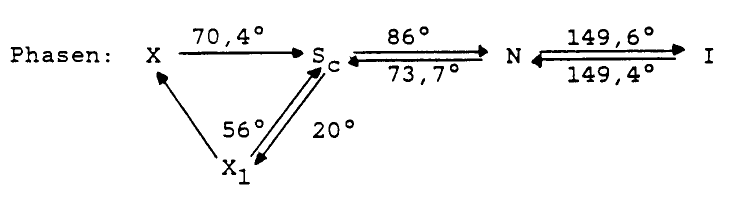

### Beispiel 23

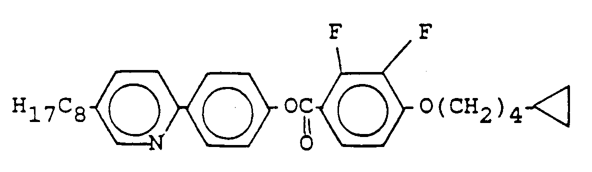

4-(4-Cyclopropylbutyloxy)-2,3-difluor-benzoesäure[4-(5-octyl-pyridin-2-yl)phenyl]ester

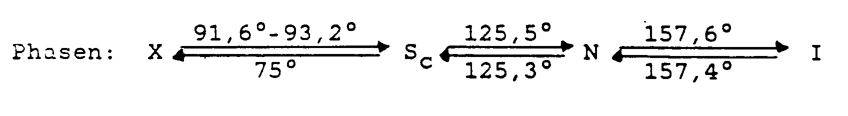

### Beispiel 24

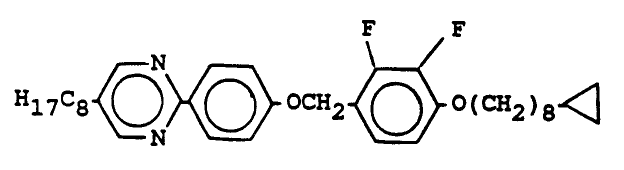

4-(8-Cyclopropyloctyloxy)-2,3-difluor-benzoesäure[4-(5-octyl-pyrimidin-2-yl)phenyl]ester

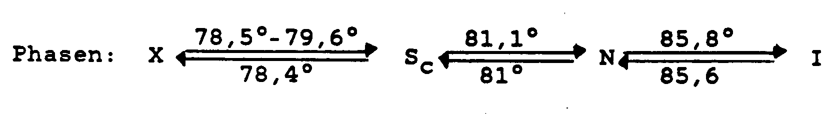

### Beispiel 25

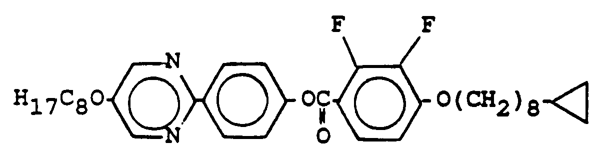

4-(8-Cyclopropyloctyloxy)-2,3-difluor-benzoesäure[4-(5-octyloxy-pyrimidin-2-yl)phenyl]ester

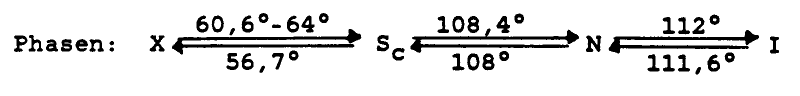

### Beispiel 26

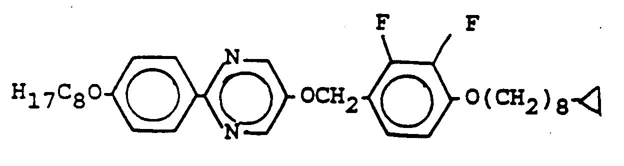

4-(8-Cyclopropyloctyloxy)-2,3-difluor-benzyl[2-(4-octyloxyphenyl)pyrimidin-5-yl]ether

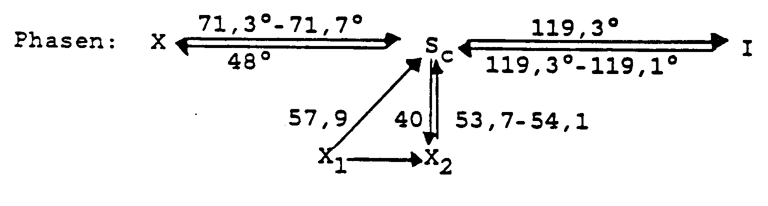

### Beispiel 27

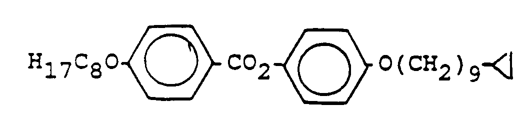

9-Cyclopropylnonyl-[4-(4-octyloxy-benzyloxy)phenyl]ether

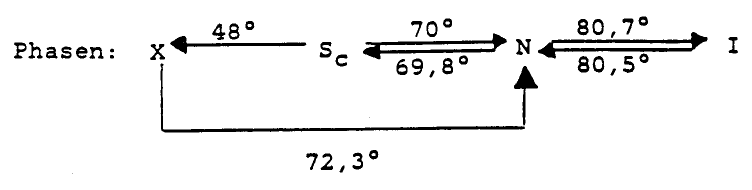

### Beispiel 28

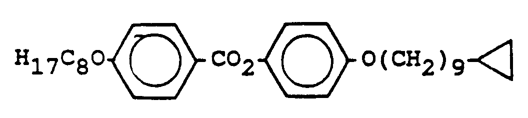

9-Cyclopropylnonyl-[4-(4-octyloxy-benzoyloxy)-benzoesäure]ester

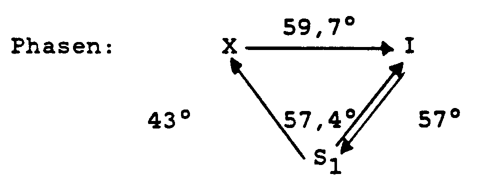

### Beispiel 29

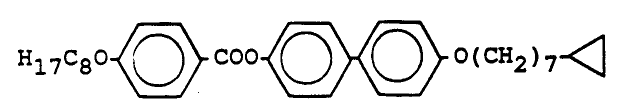

7-Cyclopropylheptyloxy[4'-(4-octyloxy-benzoyloxy)biphenyl-4-yl]

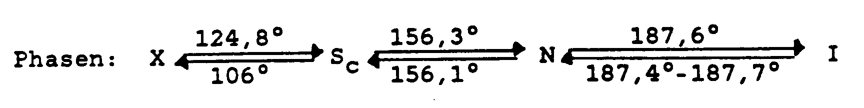

### Beispiel 30

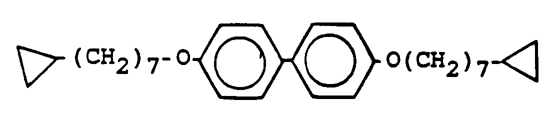

4,4'-Bis-(7-cyclopropylheptyloxy)-1,1'-biphenyl

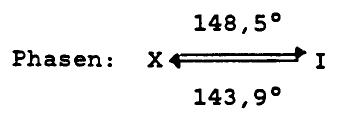

### Beispiel 31

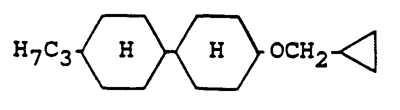

trans-4-(Cyclopropylmethyl)oxy-cyclohexyl-trans-4-propylcyclohexan 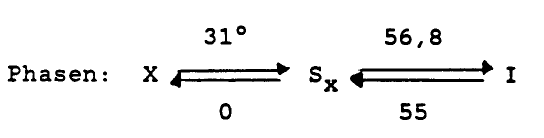
extrap. Klärpunkt ∼ 5°C

### Beispiel 32

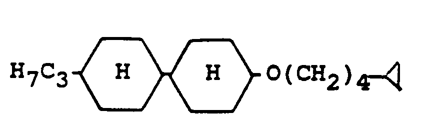

trans-4-(4-Cyclopropylbutyl)oxy-cyclohexyl-trans-4-propylcyclohexan 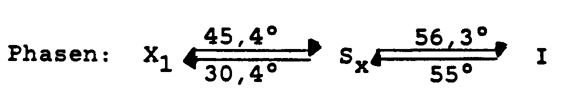
extrap. Klärpunkt ∼ 5°C

### Beispiel 33

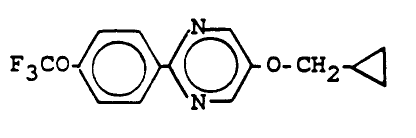

5-Cyclopropylmethyloxy-2-(4-trifluormethoxy-phenyl)pyrimidin 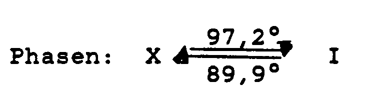
Der aus einer Mischung extrapolierte Klärpunkt liegt bei 5°C.

### Beispiel 34

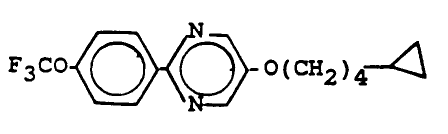

5-(4-Cyclopropylbutyl)oxy-2-(4-trifluormethoxy-phenyl)pyrimidin 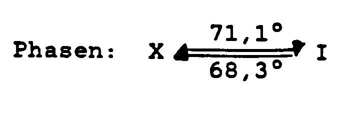
Der aus einer Mischung extrapolierte Klärpunkt liegt bei 25°C.

### Beispiel 35

Eine binäre Mischung bestehend aus:
4-Octyloxybenzoesäure-(4-hexyloxy)-phenylester 65 Mol-% und 4-(8-Cyclopropyloxtyloxy)-2,3-difluorbenzoesäure-(4-octyloxy)-phenylester (Beispiel 15) 35 Mol-% zeigt folgende Phasensequenz:
Phasen: X 34 S_{c} 63 N 76 I

### Vergleichsmischung

4-Octyloxy-benzoesäure-(4-hexyloxy)-phenylester 65 Mol-%
4-Decyloxy-2,3-difluor-benzoesäure- (4-octyloxy)phenylester 35 Mol-%
Phasen: X 37 S_{c} 69 N 81 I
Ein Vergleich der Mischung zeigt, daß die erfindungsgemäße Mischung einen niedrigeren Schmelzpunkt aufweist als die Vergleichsmischung. Die Cyclopropyl-Komponente ist daher für die praktische Anwendung besonders geeignet.

## Patentansprüche

1. Cyclopropylalkyl- oder -alkenylverbindung der allgemeinen Formel (I) 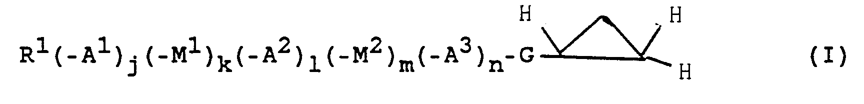 in der bedeuten:
R¹ geradkettiges oder verzweigtes (mit oder ohne asymmetrisches C-Atom) Alkyl oder Alkenyl mit 2 bis 16 C-Atomen, wobei auch eine oder zwei nicht-benachbarte -CH₂-Gruppen durch -O-, -S-, -CO-, -CO-O-, -O-CO- oder -O-CO-O- ersetzt sein können und wobei auch H durch F ersetzt sein kann, oder einer der nachfolgenden Reste 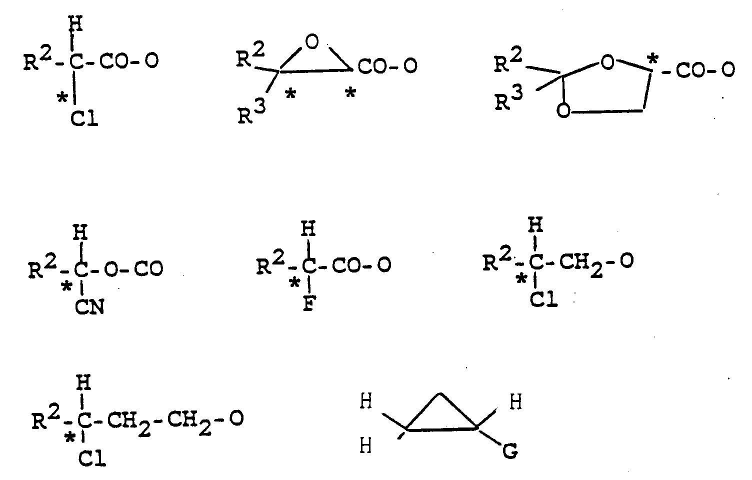 CN, OCF₃, OCF₂H, F, CF₃
A¹,A²,A³ gleich oder verschieden unsubstituiertes oder mit 1 oder 2 Halogenatomen substituiertes 1,4-Phenylen, unsubstituiertes oder in 1 oder 4 Stellung mit CN substituiertes 1,4-Cyclohexylen, Pyrazin-2,5-diyl, Pyridazin-3,6-diyl, Pyridin-2,5-diyl, Pyrimidin-2,5-diyl, (1,3,4)-Triadiazol-2,5-diyl
M¹,M² gleich oder verschieden CO-O, O-CO, CO-S, S-CO, CH₂-O, O-CH₂, C≡C, CH₂-CH₂ oder eine Einfachbindung
G geradkettiges oder verzweigtes Alkylen mit 1 bis 16 C-Atomen oder Alkenylen mit 2 bis 16 C-Atomen, bei dem auch eine oder zwei nicht-benachbarte -CH₂-Gruppen durch -O-, -S-, -O-CO-, -CO-O-, -S-CO- oder -CO-S- ersetzt sein können
R²,R³ H oder geradkettiges oder verzweigtes Alkyl mit 1 bis 16 oder Alkenyl mit 2 bis 16 C-Atomen, bei dem auch eine -CH₂-Gruppe durch -O-, -CO-O- oder -O-CO- ersetzt sein kann
k,m Null oder 1
j,l,n Null, 1 oder 2, wobei die Summe aus j, l und n 2, 3 oder 4 sein muß
wobei solche Verbindungen ausgenommen sind, bei denen
A¹,A² A³ gleich oder verschieden unsubstituiertes 1,4-Phenylen, unsubstituiertes 1,4-Cyclohexylen, Pyrazin-2,5-diyl, Pyridazin-3,6-diyl, Pyridin-2,5-diyl, Pyrimidin-2,5-diyl, (1,3,4)-Thiadiazol-2,5-diyl bedeuten und mindestens einer der Reste A¹, A², A³ nicht 1,4-Phenylen oder 1,4-Cyclohexylen ist.

2. Ausführungsform nach Anspruch 1, dadurch gekennzeichnet, daß in der allgemeinen Formel (I) die Gruppierung (-A¹)ⱼ(-M¹)ₖ(-A²)ₗ(-M²)ₘ(-A³)ₙ-bedeutet:

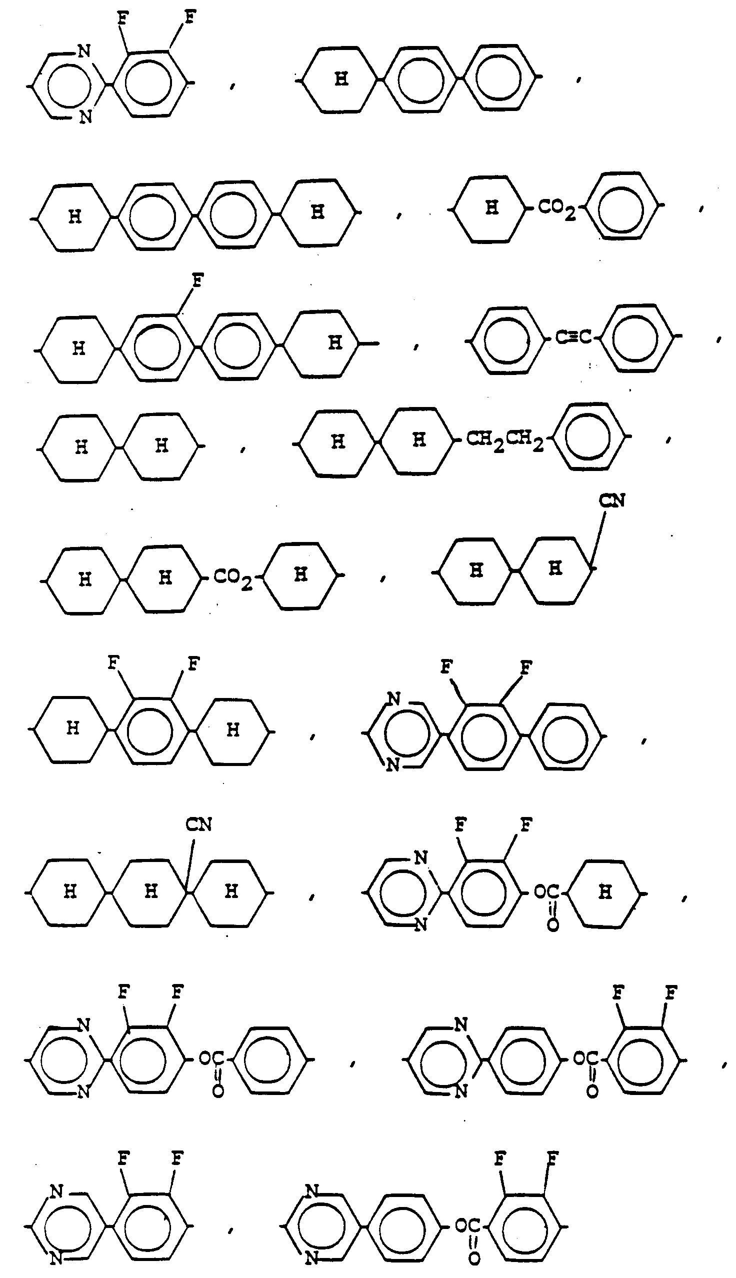

3. Ausführungsform nach Anspruch 1, dadurch gekennzeichnet, daß in der allgemeinen Formel (I) die Gruppierung R¹(A-¹)ⱼ (-M¹)ₖ (-A²)ₗ (-M²)ₘ (-A³)ₙ bedeutet:

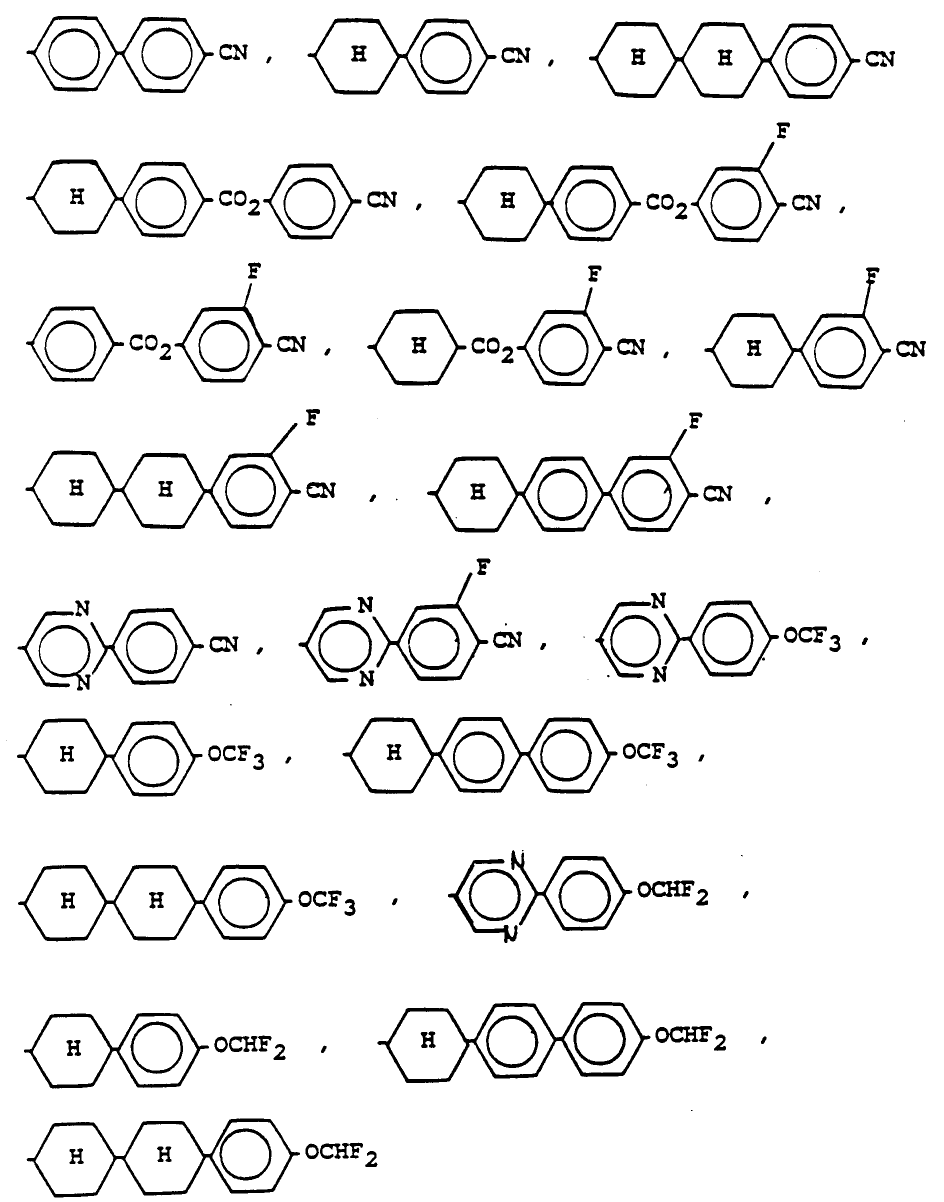

4. Ausführungsform nach den Ansprüche 1, 2 oder 3, dadurch gekennzeichnet, daß in der allgemeinen Formel (I) A¹, A² und A³ folgende Bedeutung haben:
A¹, A², A³ gleich oder verschieden mit 1 oder 2 Fluoratomen substituiertes 1,4-Phenylen, unsubstituiertes 1,4-Phenylen, 1,4-Cyclohexylen, Pyridin-2,5-diyl oder Pyrimidin-2,5-diyl.

5. Verfahren zur Herstellung der Cyclopropylalkyl- oder -alkenylverbindungen der allgemeinen Formel (I) nach Anspruch 1, 2 oder 3, dadurch gekennzeichnet, daß mesogene monofunktionell-reaktionsfähige Grundkörper der allgemeinen Formel (II) mit monofunktionell-reaktionsfähigen Cyclopropylalkyl-Verbindungen der allgemeinen Formel (III) umgesetzt werden,
R¹(-A¹)ⱼ(-M¹)ₖ(-A²)ₗ(-M²)ₘ(-A³)ₙ-X (II) 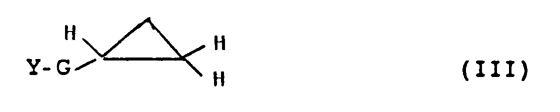
wobei X OH, O-Alkali, COOH oder COO-Alkali und Y bei der Reaktion abgehende Substituenten wie H, OH, Alkali, Halogen, Toluolsulfonyloxy oder Methylsulfonyloxy bedeuten.

6. Verfahren zur Herstellung der Cyclopropylalkyl- oder -alkenyl-Verbindungen der allgemeinen Formel (I) nach Anspruch 1, 2 oder 3 durch eine Simmons-Smith-Reaktion ausgehend von Olefinen.

7. Verwendung der Cyclopropylalkyl- oder -alkenyl-Verbindungen der allgemeinen Formel (I) nach Anspruch 1, 2 oder 3 als Komponente in Flüssigkristallmischungen.

8. Verwendung nach Anspruch 7, dadurch gekennzeichnet, daß die Flüssigkristallmischung nematisch ist.

9. Verwendung nach Anspruch 7, dadurch gekennzeichnet, daß die Flüssigkristallmischung smektisch, insbesondere chiral-smektisch, bevorzugt ferroelektrisch ist.

10. Flüssigkristalline Mischung enthaltend mindestens einen Cyclopropylalkyl- oder -alkenyl-Verbindung der allgemeinen Formel (I) nach Anspruch 1, 2 oder 3.

11. Elektrooptisches Bauteil enthaltend eine flüssigkristalline Mischung nach Anspruch 10.

## Claims

1. A cyclopropylalkyl or -alkenyl compound of the formula (I) 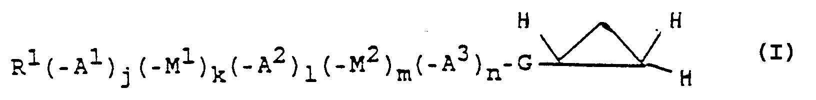 in which:
R¹ is straight-chain or branched (with or without an asymmetric carbon atom) alkyl or alkenyl having 2 to 16 carbon atoms, it also being possible for one or two non-adjacent -CH₂- groups to be replaced by -O-, -S-, -CO-, -CO-O-, -O-CO- or -O-CO-O-and it also being possible for H to be replaced by F,
or is one of the following radicals 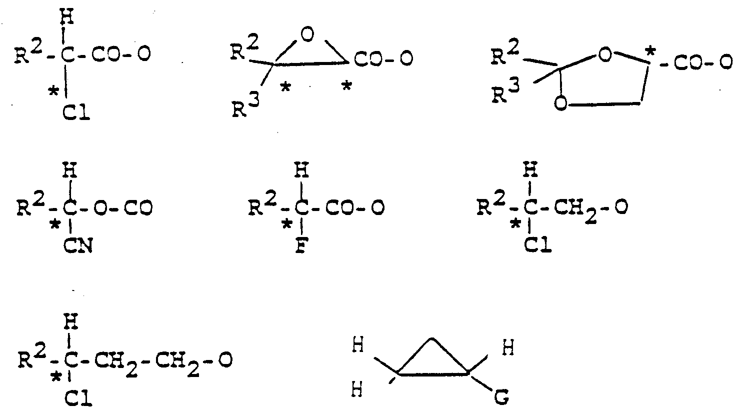 CN, OCF₃, OCF₂H, F or CF₃
A¹, A² and A³ are identical or different unsubstituted or mono- or dihalo-substituted 1,4-phenylene, unsubstituted or 1- or 4-CN-substituted 1,4-cyclohexylene, pyrazine-2,5-diyl, pyridazine-3,6-diyl, pyridine-2,5-diyl, pyrimidine-2,5-diyl or (1,3,4)-thiadiazole-2,5-diyl
M¹ and M² are identical or different CO-O, O-CO, CO-S, S-CO, CH₂-O, O-CH₂, C≡C, CH₂-CH₂ or a single bond
G is straight-chain or branched alkylene having 1 to 16 carbon atoms or alkenylene having 2 to 16 carbon atoms in which it is also possible for one or two non-adjacent -CH₂- groups to be replaced by -O-, -S-, -O-CO-, -CO-O-, -S-CO- or -CO-S-
R² and R³ are H or straight-chain or branched alkyl having 1 to 16 carbon atoms or alkenyl having 2 to 16 carbon atoms in which it is also possible for one -CH₂- group to be replaced by -O-, -CO-O- or -O-CO-
k and m are zero or 1 and
j, l and n are zero, 1 or 2, where the sum of j, l and n must be 2, 3 or 4
those compounds being excluded in which
A¹, A² and A³ are identical or different unsubstituted 1,4-phenylene, unsubstituted 1,4-cyclohexylene, pyrazine-2,5-diyl, pyridazine-3,6-diyl, pyridine-2,5-diyl, pyrimidine-2,5-diyl or 1,3,4-thiadiazole-2,5-diyl and at least one of the radicals A¹, A² and A³ is not 1,4-phenylene or 1,4-cyclohexylene

2. An embodiment as claimed in claim 1, wherein, in the formula (I), the group
(-A¹)ⱼ(-M¹)ₖ(-A²)ₗ(-M²)ₘ(-A³)ₙ- is:

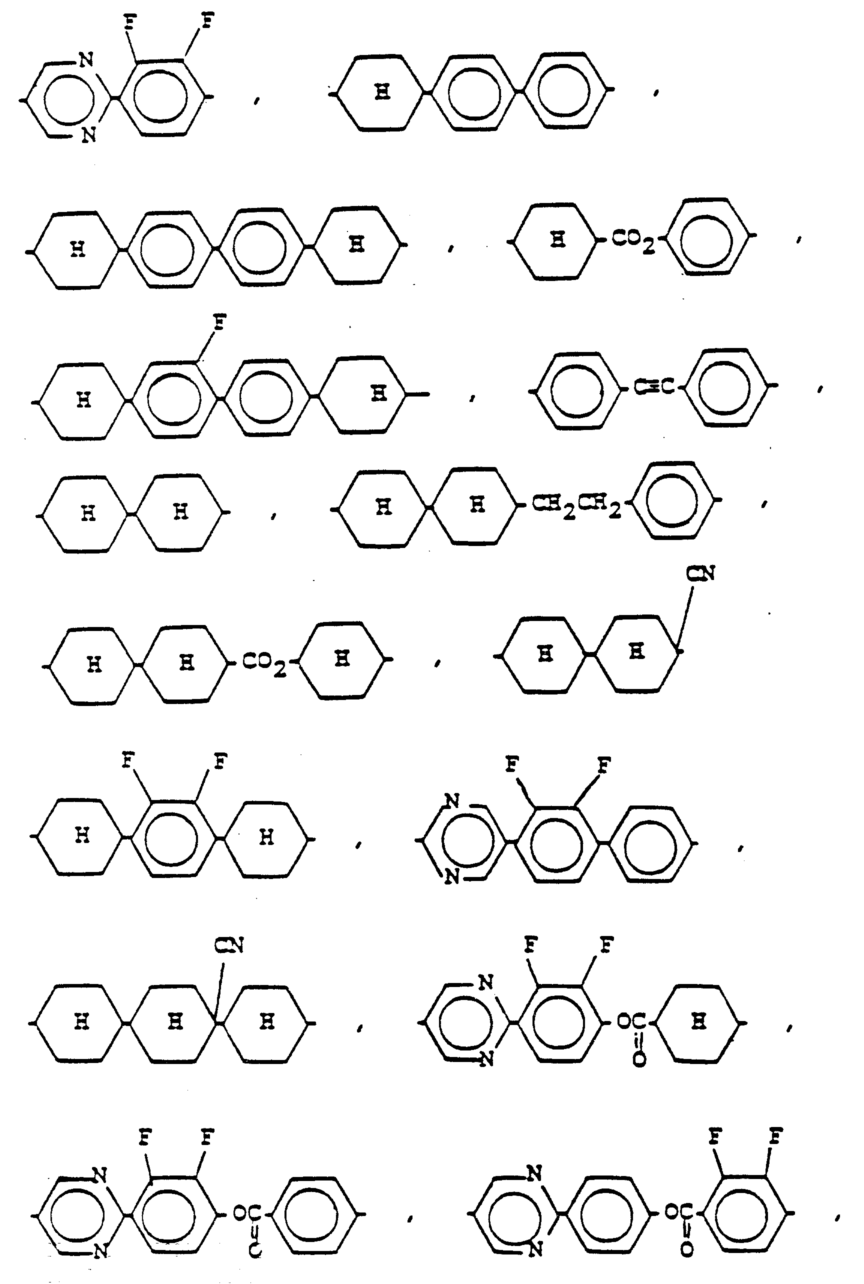

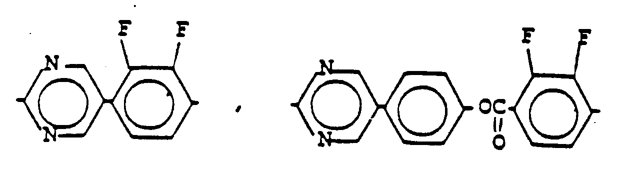

3. An embodiment as claimed in claim 1, wherein, in the formula (I), the group R¹(A-¹)ⱼ(-M¹)ₖ(-A²)ₗ(-M²)ₘ (-A³)ₙ is:

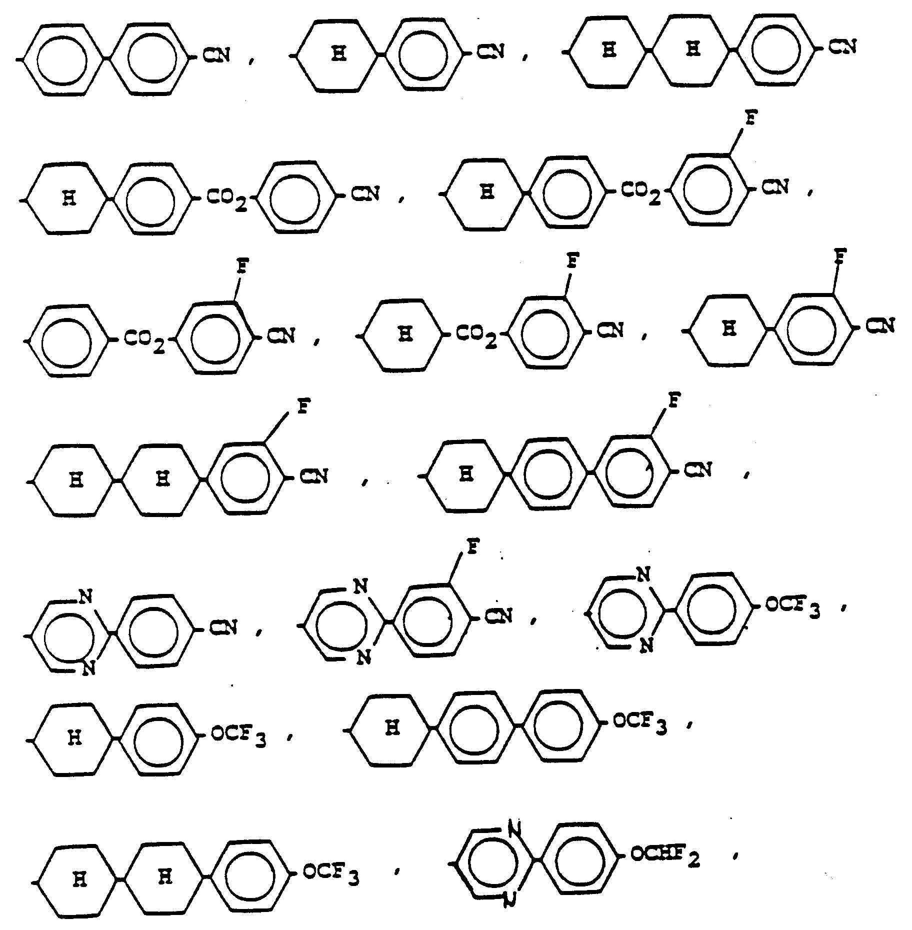

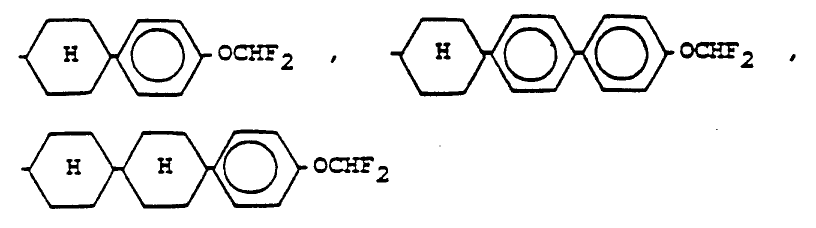

4. An embodiment as claimed in claims 1, 2 or 3, wherein, in the formula (I), A¹, A² and A³ have the following meaning:
A¹, A² and A³ are identical or different mono- or difluoro-substituted 1,4-phenylene, unsubstituted 1,4-phenylene, 1,4-cyclohexylene, pyridine-2,5-diyl or pyrimidine-2,5-diyl.

5. A process for the preparation of a cyclopropylalkyl or -alkenyl compound of the formula (I) as claimed in claim 1, 2 or 3, which comprises reacting a mesogenic monofunctionally reactive parent compound of the formula (II) with a monofunctionally reactive cyclopropylalkyl compound of the formula (III)
R¹(-A¹)ⱼ(-M¹)ₖ(-A²)ₗ(-M²)ₘ(-A³)ₙ-X (II) 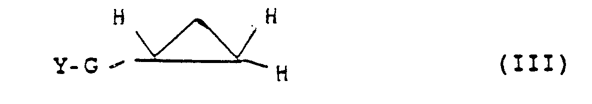
where X is OH, O-(alkali metal), COOH or COO-(alkali metal), and Y is a substituent which is released during the reaction, such as H, OH, alkali metal, halogen, toluenesulfonyloxy or methylsulfonyloxy.

6. A process for the preparation of a cyclopropylalkyl or -alkenyl compound of the formula (I) as claimed in claim 1, 2 or 3 by a Simmons-Smith reaction starting from an olefin.

7. The use of a cyclopropylalkyl or -alkenyl compound of the formula (I) as claimed in claim 1, 2 or 3 as a component in liquid-crystal mixtures.

8. The use as claimed in claim 7, wherein the liquid-crystal mixture is nematic.

9. The use as claimed in claim 7, wherein the liquid-crystal mixture is smectic, in particular chiralsmectic, preferably ferroelectric.

10. A liquid-crystalline mixture containing at least one cyclopropylalkyl or -alkenyl compound of the formula (I) as claimed in claim 1, 2 or 3.

11. An electrooptical component containing a liquid-crystalline mixture as claimed in claim 10.

## Revendications

1. Composé de cyclopropylalkyle ou de cyclopropylalcényle de formule générale (I) 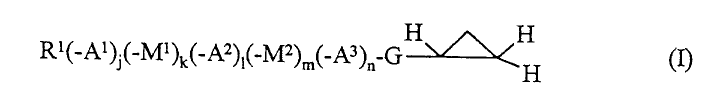 dans laquelle
R¹ représente un reste alkyle ou alcényle linéaire ou ramifié (avec ou sans atome de carbone asymétrique) de 2 à 16 atomes de carbone, dans lequel un ou deux groupes -CH₂- non adjacents peuvent être remplacés par -O-, -S-, -CO-, -CO-O-, -O-CO- ou -O-CO-O-, et des H peuvent être remplacés par des F, ou l'un des restes suivants: 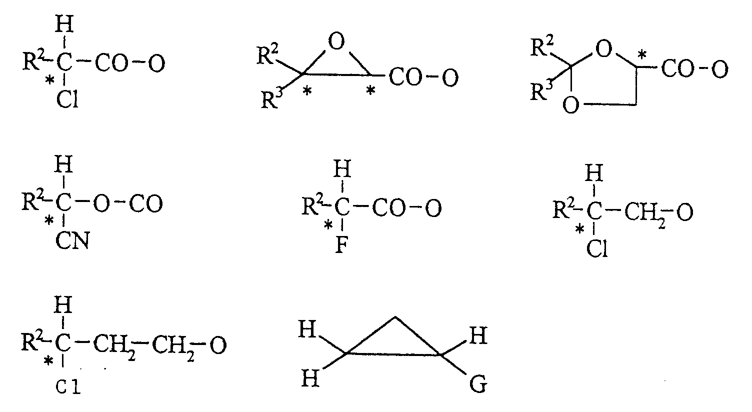 CN, OCF₃, OCF₂H, F, CF₃;
A¹, A², A³ sont identiques ou différents et représentent un reste 1,4-phénylène non substitué ou substitué par 1 ou 2 atomes d'halogène, 1,4-cyclohexylène non substitué ou substitué en position 1 ou 4 par CN, pyrazine-2,5-diyle, pyridazine-3,6-diyle, pyridine-2,5-diyle, pyrimidine-2,5-diyle, (1,3,4)-thiadiazole-2,5-diyle;
M¹, M² sont identiques ou différents et représentent des groupes CO-O, O-CO, CO-S, S-CO, CH₂-O, O-CH₂, C≡C, CH₂-CH₂ ou une simple liaison;
G représente un reste alkylène de 1 à 16 atomes de carbone ou alcénylène de 2 à 16 atomes de carbone, linéaire ou ramifié, dans lequel un ou deux groupes CH₂ non adjacents peuvent être remplacés par -O-, -S-,-O-CO-, -CO-O-, -S-CO- ou -CO-S-;
R², R³ représentent un atome d'hydrogène ou un reste alkyle de 1 à 16 atomes de carbone ou alcényle de 2 à 16 atomes de carbone, linéaire ou ramifié, dans lequel un groupe CH₂ peut être remplacé par -O-, -CO-O- ou -O-CO-;
k , m sont 0 ou 1;
j, l, n représentent 0, 1 ou 2, la somme de j, l et n devant être égale à 2, 3 ou 4,
à l'exception des composés dans lesquels
A¹,A²,A³ sont indentiques ou différents et représentent des restes 1,4-phénylène non substitué, 1,4-cyclohexylène non substitué, pyrazine-2,5-diyle, pyrezidinecyclohexylène non substitué, pyrazine-2,5-diyle, pyridazine-3,6-diyle, pyridine-2,5-diyle, pyrimidine-2,5-diyle, (1,3,4)-thiadiazole-2,5-diyle et au moins l'un des restes A¹, A², A³ n'est pas un reste 1,4-phénylène ou 1,4-cyclohexylène.

2. Mode de réalisation selon la revendication 1, caractérisé en ce que, dans la formule générale (I), le groupement (-A¹)ⱼ(-M¹)ₖ(-A²)ₗ(-M²)ₘ(-A³)ₙ- représente

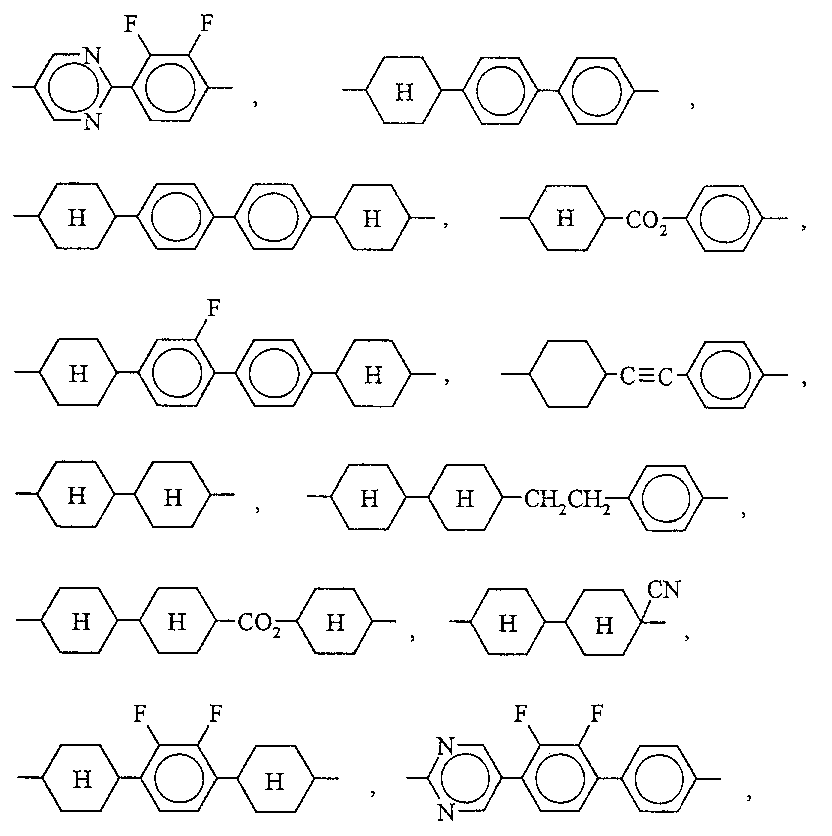

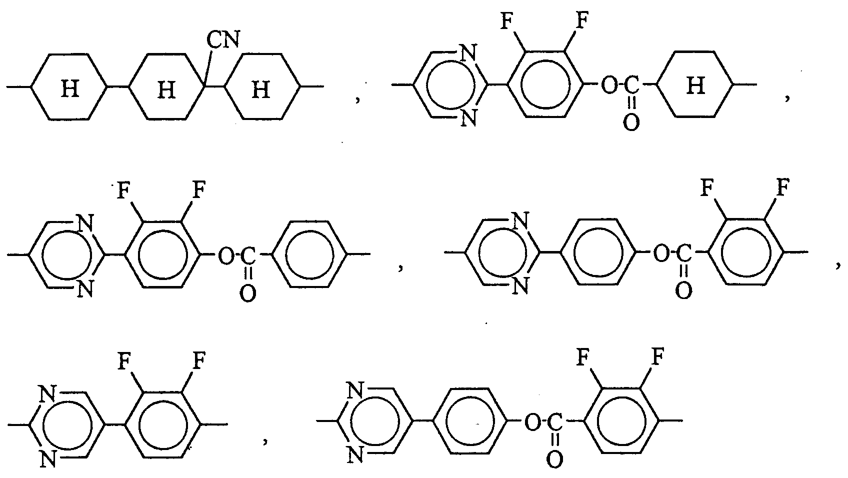

3. Mode de réalisation selon la revendication 1 caractérisé en ce que, dans la formule générale (I), le groupement R¹(-A¹)ⱼ(-M¹)ₖ(-A²)ₗ(-M²)ₘ(-A³)ₙ représente

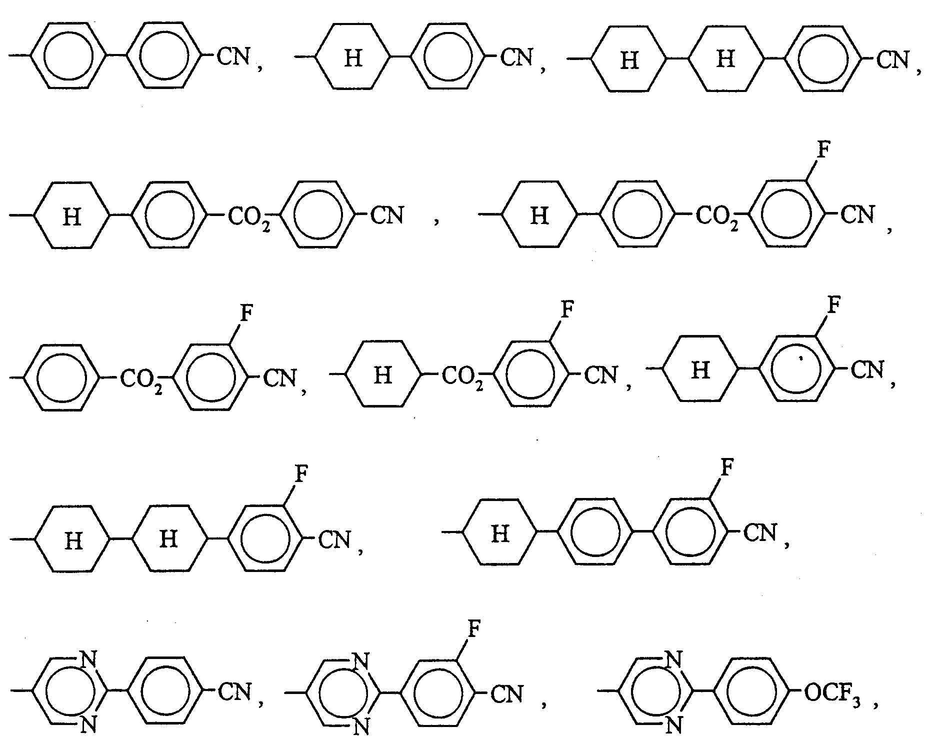

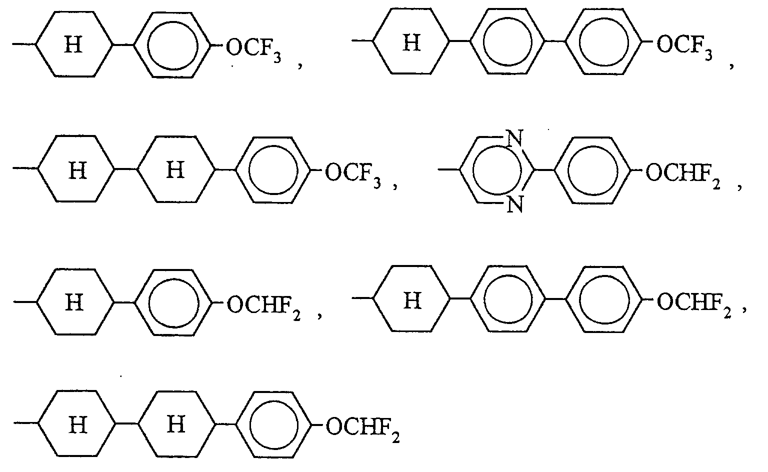

4. Mode de réalisation selon les revendications 1, 2 ou 3, caractérisé en ce que, dans la formule générale (I), A¹, A² et A³ ont la signification suivante:
A¹, A², A³ sont identiques ou différents et représentent des restes 1,4-phénylène substitué par 1 ou 2 atomes de fluor, 1,4-phénylène non substitué, 1,4-cyclohexylène, pyridine-2,5-diyle ou pyrimidine-2,5-diyle.

5. Procédé de préparation des composés de cyclopropylalkyle ou de cyclopropylalcényle de formule générale (I) selon la revendication 1, 2 ou 3, caractérisé en ce que l'on fait réagir des composés de base mésogènes de formule générale (II), ayant un seul groupe fonctionnel réactif, avec des composés de cyclopropylalkyle de formule générale (III) ayant un seul groupe fonctionnel réactif,
R¹(-A¹)ⱼ(-M¹)ₖ(-A²)ₗ(-M²)ₘ(-A³)ₙ-X (II) 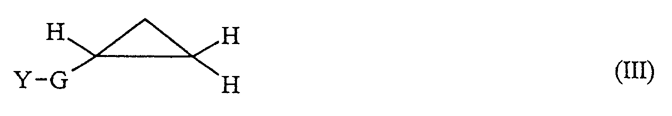
X étant OH, O-métal alcalin, COOH ou COO-métal alcalin et Y étant un substituant partant dans la réaction tel que H, OH, un métal alcalin, un halogène, un toluènesulfonyloxy ou un méthylsulfonyloxy.

6. Procédé de préparation des composés de cyclopropylalkyle ou de cyclopropylalcényle de formule générale (I) selon la revendication 1, 2 ou 3, à partir d'oléfines par une réaction de Simmons-Smith.

7. Utilisation des composés de cyclopropylalkyle ou de cyclopropylalcényle de formule générale (I) selon la revendication 1, 2 ou 3 comme constituants de mélanges de cristaux liquides.

8. Utilisation selon la revendication 7, caractérisée en ce que le mélange de cristaux liquides est nématique.

9. Utilisation selon la revendication 7, caractérisée en ce que le mélange de cristaux liquides est smectique, en particulier smectique chiral, de préférence ferro-électrique.

10. Mélange de cristaux liquides contenant au moins un composé de cyclopropylalkyle ou de cyclopropylalcényle de formule générale (I) selon la revendication 1, 2 ou 3.

11. Elément électro-optique contenant un mélange de cristaux liquides selon la revendication 10.
